Europäisches Patentamt

European Patent Office

Office européen des brevets

(19)

(11) Publication number: **0 228 726 B1**

## EUROPEAN PATENT SPECIFICATION

(12)

(45) Date of publication of patent specification: 03.04.91

(21) Application number: 86202061.7

(22) Date of filing: 20.11.86

(51) Int. Cl.⁵: **C12N 15/11, C12N 15/74, C12N 1/21, C12P 21/02, //(C12N1/21,C12R1:07,1:19, 1:46)**

(54) Method for preparing proteins using transformed lactic acid bacteria.

(30) Priority: 29.11.85 NL 8503316

(43) Date of publication of application:
15.07.87 Bulletin 87/29

(45) Publication of the grant of the patent:
03.04.91 Bulletin 91/14

(84) Designated Contracting States:
BE DE FR NL

(56) References cited:
WO-A-85/03945

APPLIED AND ENVIRONMENTAL MICROBI-
OLOGY, vol. 48, no. 4, October 1984, pages
726-731, American Society for Microbiology,
US; J. KOK et al.: "Construction of plasmid
cloning vectors for lactic Streptococci
which also replicate in Bacillus subtilis and
Escherichia coli"

(73) Proprietor: STICHTING NEDERLANDS IN-
STITUUT VOOR ZUIVELONDERZOEK
Kernhemseweg 2 P.O.Box 20
NL-6710 BA Ede(NL)

(72) Inventor: De Vos, Willem Meindert
Nettelhorst 26
NL-6714 MD Ede(NL)
Inventor: Simons, Augustinus Franciscus Ma-
ria
Durendaal 17
NL-6715 JP Ede(NL)

(74) Representative: van der Beek, George Frans,
Ir. et al
Nederlandsch Octrooibureau Schevening-
seweg 82 P.O. Box 29720
NL-2502 LS 's-Gravenhage(NL)

APPLIED AND ENVIRONMENTAL MICROBI-
OLOGY, vol. 50, no. 2, August 1985, pages
540-542, American Society for Microbiology,
US; J.M.B.M. VAN DER VOSSEN et al.:
"Construction of cloning, promoter-
screening, and terminator-screening shuttle
vectors for Bacillus-subtilis and Streptococ-
cus lactis"

APPLIED AND ENVIRONMENTAL MICROBI-
OLOGY, vol. 50, no. 1, July 1985, pages
94-101, American Society for Microbiology,
US; J. KOK et al.: "Cloning and expression of
a Streptococcus cremoris proteinase in Ba-
cillus subtilis and Streptococcus lactis"

APPLIED AND ENVIRONMENTAL MICROBI-
OLOGY, vol. 49, no. 1, January 1985, pages
115-119, American Society for Microbiology;
MY LIEN DAO et al.:
"Streptococcus-Escherichia coli shuttle vec-
tor pSA3 and its use in the cloning of Strep-
tococcal genes"

NETH. MILK DAIRY J., vol. 40, no. 2/3, 1986,
pages 141-154; W.M. DE VOS: "Gene cloning
in lactic Streptococci"

APPLIED AND ENVIRONMENTAL MICROBI-
OLOGY, vol. 48, no. 2, August 1984, pages
252-259, American Society for Microbiology;
J.K. KONDO et al.: "Plasmid transformation
of Streptococcus lactis protoplasts: optimiz-
ation and use in molecular cloning"

FEMS Microbiology Letters, vol. 30(1985), pp.
193-196

# EP 0 228 726 B1

**Description**

The invention relates to the application of recombinant DNA technology with the aim of producing in an efficient manner polypeptides in bacteria, in particular in lactic acid bacteria, in the bacterium B. subtilis and in the bacterium E. coli . More particularly the invention relates to the provision of plasmid vectors which replicate in both Gram-negative and Gram-positive bacteria, including lactic acid bacteria, as host organisms, and which are suitable for cloning and bringing genes to expression in said bacteria, in particular a phospho-β-galactosidase gene. The invention relates very particularly to the provision of DNA fragments or plasmid vectors with a replicon, a promoter, a ribosome binding site and a gene which advantageously originates from lactic acid bacteria and which functions in lactic acid bacteria, in B. subtilis and in E. coli , and also to a method of preparation for these. The invention also relates to a method for cloning genes in lactic acid bacteria. Finally the invention embraces the host organisms which are provided with one or more plasmid vectors according to the invention.

The development and the application of recombinant DNA technology in genetic model systems such as the Gram-negative E. coli and the Gram-positive B. subtilis has shown that bacteria are capable of producing foreign (heterologous) polypeptides in an efficient manner and, in addition, can be stimulated to an increased or reduced production of species-intrinsic (homologous) polypeptides. The basic elements for the application of said recombinant DNA technology are:

1. a vector, generally a plasmid, into which homologous or heterologous genes can be introduced in order to achieve stable replication and high expression of said genes. A vector contains one or more DNA fragments which ensure a stable replication (replicon) and, in order to simplify genetic selection, is often endowed with easily selectable genes such as antibiotic-resistance genes;

2. a DNA fragment containing a gene which codes for the required property, which may be a species-intrinsic (homologous) or a foreign (heterologous) property, which DNA fragment is provided with a suitable controlling DNA sequence;

3. one or more DNA fragments containing controlling DNA sequences which are necessary for expression in the host organism, such as a promoter (a DNA sequence which ensures efficient transcription of the gene to be brought to expression) and a ribosome binding site (a DNA sequence which initiates the efficient translation of the transcript formed);

4. a suitable host bacterium in which the vector containing the required gene having the required controlling DNA sequence can be transformed and can be stably maintained, and which possesses the cellular systems to bring the gene concerned to expression.

The application of recombinant DNA technology has led to the production of polypeptides which possess enzymatic activity and catalyse biological conversions such as α-amylase and glucose-isomerase, or which find pharmaceutical application, such as insulin and interferon. Auxiliary substances for the foodstuffs industry such as chymosin, β-galactosidase and β-glucanase, can apparently be produced by the use of recombinant DNA technology.

For the application of recombinant DNA technology in bacteria, mainly E. coli , and to a lesser extent B. subtilis have hitherto been used as host and production organisms. The most important reason for this is that said bacteria are genetically best characterized and are used as model systems for Gram-negative (E. coli ) and Gram-positive (B. subtilis )bacteria. All the initial discoveries and inventions of recombinant DNA technology were made using E. coli as host. However, E. coli and B. subtilis are not the most suitable bacteria for the commercial production of polypeptides for use in the foodstuffs industry: viz., E. coli may produce toxic and pyrogenic compounds and is therefore not permissible in foodstuffs, while B. subtilis is a very active producer of protease, as a result of which the yield of most of the polypeptides produced is very considerably reduced. For nutrient fermentations, which generally proceed in an anaerobic medium, said bacteria are either totally unsuitable (B. subtilis : obligate aerobe) or less suitable (E. coli ). Moreover, the application of both types of bacteria is considerably limited in the foodstuffs industry because the taste resulting from the presence of said bacteria is found to be especially unpleasant. This is a considerable drawback especially for application in dairy products: contaminations with B. subtilis or E. coli bring about unacceptable changes in taste.

Lactic acid bacteria have already been used for thousands of years in the preparation of fermented foodstuffs and consumed in large quantities; these bacteria can safely be used. In addition, lactic acid bacteria make an important contribution to the consistency and the taste of said products, and large-scale anaerobic fermentations by means of these microaerophilic bacteria are possible in a simple manner. Lactic acid bacteria are therefore pre-eminently suitable as Gram-positive host and production organisms in the application of recombinant DNA technology in the foodstuffs industry.

The mesophilic lactic acid bacteria belonging to the genera Streptococcus lactis and S. cremoris are

3

used in the preparation of a great variety of dairy products such as cheese, curd cheese, butter and buttermilk. It has emerged that said lactic acid bacteria generally have a plasmid complement which consists of various plasmids varying in number from one to more than six, with molecular weights from approximately $10^6$ to over $100 \times 10^6$. Although most of these plasmids have no known function (so-called cryptic plasmids) it has been shown that in a number of cases important functions such as lactose and citrate metabolism, proteinase production, bacteriocine production and resistance and bacteriophage resistance mechanisms are wholly or partly coded by plasmid DNA.

By analogy with host-vector systems for the genetic model bacteria E. coli and B. subtilis , endogenous plasmids can be used for the construction of vectors for lactic acid bacteria. An example of this is the recently described vector pGK12 based on the replicon of the S. cremoris plasmid pWV01. Said vector replicates in S. lactis , B. subtilis and E. coli . Said vector is, however, less suitable for the application of recombinant DNA technology in lactic acid bacteria because the copy number of said vector in the lactic acid bacterium S. lactis is very low (approximately four copies per cell; Appl. Environ. Microbiol. 48 (1984) 726-731). Another example is the vector pGB301 which is made up of an S. sanguis replicon and which appears to replicate in the lactic acid bacterium S. lactis (Appl. Environ. Microbiol. 48 (1984) 252-259). The drawback of said vector is also the relatively low copy number and the large size (9.8 kb; Plasmid 4 (1938) 130-138).

It is known from the results which have hitherto been obtained using recombinant DNA technology that the level of gene expression of cloned genes in bacterial systems is strongly related to the copy number of said genes and therefore to the copy number of the vectors used for the cloning. Vectors which replicate with a substantial copy number in S.lactis are indicated in summarized form in FEMS Microbiol. Lett. Vol.30 (1985), pages 193-195. This reference discloses the vectors pCK1 and pCK21 having copy numbers in S. lactis of 54 and 60 respectively. These vectors carry the replicon of the streptococcal plasmid pSH71 and replicate in E.coli , B.subtilis and S.lactis .

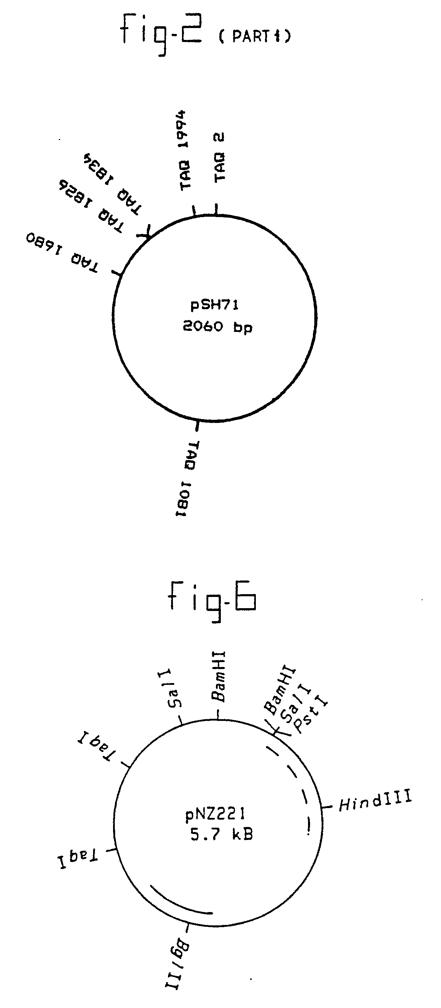

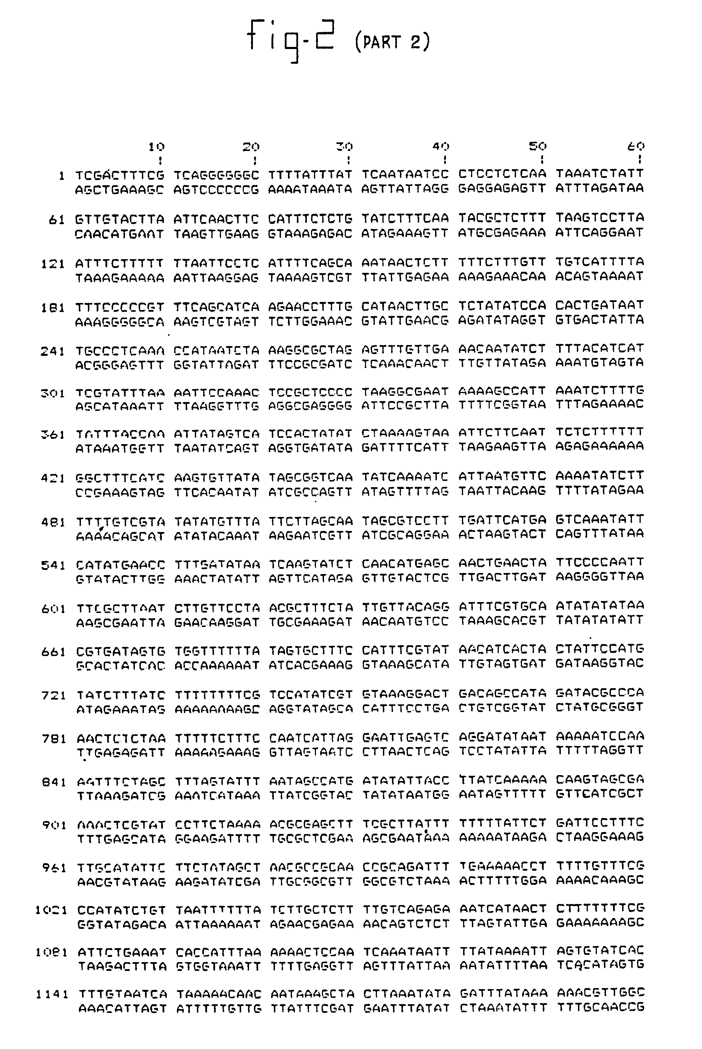

This invention relates to a vector with a small size which exhibits a high copy number in lactic acid bacteria and to a method which leads to the construction of such a vector. More in particular the invention is related - inter alia - to DNA fragments coding for (a) a replicon activity in lactic acid bacteria and (b) at least one antibiotic-resistance gene and characterized in that the DNA fragment coding for the replicon activity has the DNA sequence extending from base pair 2 to base pair 1680 of the sequence shown in Figure 2, parts 2 and 3 or a mutated form thereof having the same activity.

The method which has led to the construction of such a vector comprises a so-called replicon screening strategy which makes use of the model organism B. subtilis as an intermediate host for the initial selection of DNA fragments with replicon activity. This use is based on the fact that B. subtilis can be transformed with a much higher frequency than lactic acid bacteria (Mol. Gen. Genet. 168 (1979) 111-115; Appl. Environ. Microbiol. 43 (1982) 1213-1215) and on the likeness which obviously exists between said Gram-positive organisms in relation to replicon activity since the lactic acid bacterium plasmid pWV01 originating from S. cremoris appears to replicate in B. subtilis (Lactic Acid Bacteria in Foods, published by Neth. Soc. Micro-biol. (1983) p. 33).

An important advantage of said method is, moreover, that each constructed vector will replicate in B. subtilis , which increases the gene manipulation possibilities.

An essential component of the replicon screening strategy described here is the development of a plasmid which replicates not only in B. subtilis or lactic acid bacteria but in E. coli and, in addition, has Gram-positive antibiotic-resistance genes which are situated on a suitable and small restriction DNA fragment and which are expressed in E. coli , B. subtilis and, as expected, in lactic acid bacteria as well (replicon screening vector). The ligation of said restriction fragment, which contains the antibiotic-resistance genes, with restriction fragments of plasmids originating from and replicating in lactic acid bacteria followed by transformation of B. subtilis may result in antibiotic-resistant transformants. The plasmid DNA isolated from said transformants is then transformed into plasmid-free or plasmid-containing lactic acid bacteria, and antibiotic-resistant lactic acid bacteria are selected. The structure and the copy number of the plasmids in the transformed lactic acid bacteria found are then determined by means of standard techniques. By using the method described above with the replicon screen vector pNZ10 screening, a number of new vectors has been developed which are capable of transforming a lactic acid bacterium. A special case of this is the vector pNZ12 having a size of 4.1 kb, which is provided with genes which bring about resistance to the antibiotics chloramphenicol and kanamycin and which replicates and is maintained with a high copy number (approximately 100 copies per cell) in lactic acid bacteria. In addition to replication in lactic acid bacteria and in B. subtilis , the vector pNZ12 is also capable of transforming the Gram-negative model bacterium E. coli . Moreover, the unique Sal I and Bgl II sites in the vector are of importance in cloning experiments in said hosts. At these sites it is possible to insert in DNA fragments which, after transforming the recombinant plasmids into lactic acid bacteria, continue to be maintained in the required lactic acid bacterium with a high copy number. A special case occurs on inserting DNA into the Bgl II site which is situated in the kanamycin-resistance gene. As a result of this, this marker is inactivated, which makes simple genetic selection possible (insertion inactivation; W.M. de Vos, academic thesis of the State University of Groningen, 1983, Bariet, Ruinen).

Direct gene cloning by means of shotgun experiments in lactic acid bacteria is impeded to a considerable degree by the absence of an efficient transformation system. Although the transformation frequencies which were initially reached in S. lactis (Appl. Environ. Microbiol. 43 (1982) 1212-1215) have been increased by some technical modifications by a factor of ten to approximately $10^3$ transformants per μg.(Appl. Environ. Microbiol. 48 (1984) 252-259), an optimized transformation system as described for E. coli or B. subtilis ($10^7$ transformants per μg) has not been achieved for lactic acid bacteria. Gene clonings in lactic acid bacteria will therefore generally have to be carried out by using intermediate hosts. The vector pNZ12 described here replicates both in E. coli and B. subtilis and in lactic acid bacteria and is therefore extremely suitable for using the genetically readily accessible hosts E. coli and B. subtilis in gene clonings aimed at lactic acid bacteria. The use described above of intermediate hosts in gene cloning in lactic acid bacteria, with the use of E. coli and B. subtilis by means of the vector pNZ12 as a preferred embodiment, is a component of the present invention. In this manner, by making use of the unique Sal I site in the vector pNZ12, a DNA fragment which originated from S. lactis and which contained the phospho-β-galactosidase gene was cloned in E. coli . Said recombinant plasmid pNZ32 was then transformed in both B. subtilis and a lactic acid bacterium. In all the said hosts the phospho-β-galactosidase gene is expressed. Said expression can be determined in a very simple manner via an enzymatic reaction, as a result of which a new possibility is presented for measuring gene expression in E. coli and B. subtilis , but in particular in lactic acid bacteria.

To bring a gene to expression in a lactic acid bacterium said gene always has to have upstream two controlling DNA elements which are functional in lactic acid bacteria: a promoter and a ribosome binding site. Genes which already have these two DNA elements (such as, in general, homologous genes) can therefore be expressed, but the level of gene expression can generally be increased if a second stronger promoter is placed upstream. Genes which are only provided with a ribosome binding site can only be brought to expression by placing a promoter upstream. Genes which have neither of the two controlling DNA elements (such as, generally, heterologous genes) can only be brought to expression if they are provided with said DNA elements. For this purpose, the gene to be brought to expression is coupled to a gene which does contain a promoter and a ribosome binding site. When the reading frames of the two genes are contiguous, a fusion protein is produced. In some cases said fusion protein may possess the required biological activity. In other cases it is sometimes possible to liberate the required protein from such a fusion protein.

The nature of the promoter and of the ribosome binding site to a large extent determines the expression of the gene situated downstream. DNA fragments on which a promoter and a ribosome binding site are located and which originate from, and function in, lactic acid bacteria, were hitherto unknown, as a result of which progress in the application of recombinant DNA technology in said bacteria was impeded to a large extent. The invention now offers a method for isolating DNA fragments specific to lactic acid bacteria, on which fragments said two controlling DNA sequences are located. The method developed for this purpose is based on the use of a so-called promoter screening vector and of E. coli and B. subtilis as intermediary hosts. Such vectors have been used successfully for cloning promoter fragments in E. coli and B. subtilis and generally consist of a vector containing an antibiotic-resistance gene which has been stripped of its own promoter and is not therefore (or is very poorly) expressed. By introducing usable unique restriction sites just upstream of the gene decapitated in this manner, the applicability of a promoter screening vector is considerably increased. Isolation of controlling DNA elements can then be carried out by inserting DNA fragments into said unique restriction sites followed by transforming the intermediary host B. subtilis or E. coli and selecting transformants in which the decapitated antibiotic-resistance gene is again brought to expression (antibiotic-resistant transformants). The DNA fragments which are used for ligating in may be derived from chromosomal DNA, plasmid DNA and bacteriophage DNA of lactic acid bacteria. It is known that powerful promoters primarily occur on bacteriophage DNA.

Application of the method described above to the promoter screening vector pNZ220, which is derived from the vector pNZ12 described above and therefore replicates in B. subtilis , in E. coli and in a lactic acid bacterium, results in the isolation of the expression plasmid pNZ221 which contains a DNA fragment originating from a lactic acid bacteriophage which has strong promoter activity in said hosts.

On closer analysis it emerged that said DNA fragment probably contains two promoters which are located immediately in sequence and which have the same orientation. Moreover, said promoters appeared

EP 0 228 726 B1

to be followed at a short distance by a ribosome binding site, a start codon (ATG), a so-called open reading frame and, finally, a number of usable restriction sites. As those who are skilled in the art will realise, said DNA fragment specific to lactic acid bacteria and the information mentioned here can be used extremely well for bringing homologous and heterologous genes to expression in lactic acid bacteria.

The vectors and derived plasmids described here are able to replicate both in lactic acid bacteria and in B. subtilis and E. coli . As a result of this, all the gene manipulation techniques which the development of recombinant DNA technology has realized and has still to realise in these model organisms and are still to be realized can be applied to said vectors and derived plasmids.

Until now in lactic acid bacteria only the transformation of S. lactis strains which have been deprived of their natural plasmid DNA content (Appl. Environ. Microbiol. 48 (1984) 252-259; 726-731) in the laboratory has been described. But natural S. lactis strains containing plasmids also appear to be transformable with the vector described here. In addition, it is possible to transfer plasmids from one lactic acid bacterium to another in an efficient manner by means of conjugation. This applies also to the vectors described here. In this manner it is possible to realize various usable plasmid combinations in lactic acid bacteria without being limited by the low transformation frequency which has hitherto been achieved with said organisms. Therefore, the fact that until now S. lactis has been the only transformable lactic acid bacterium does not rule out the possibility of other lactic acid bacteria being used as a host for the vector and derived plasmids described here. In particular S. cremoris will be a very suitable host for the application of the expression-controlling DNA elements present in the expression vector pNZ221 and originating from an S. cremoris phage.

Application of the vector described here, the expression vector derived therefrom and the cloning system for lactic acid bacteria may lead to the development of lactic acid bacteria which produce polypeptides in an efficient and stable manner. Those who are skilled in the art will realize that use of said lactic acid bacteria offers very great advantages in the foodstuffs industry. Thus, increased and stabilized production of enzymes which are a component of the proteolytic system of a lactic acid bacterium (such as proteinases and peptidases) will result in an acceleration of the development of the required taste of a diary product such as, for example, cheese. Moreover, the bacteriophage resistance of a lactic acid bacterium can be enhanced by increasing and stabilizing the expression of genes which prevent bacteriophage adsorption or by cloning and expressing restriction modification systems. Similarly, lactic acid bacteria can be developed which have the ability to produce chymosin or other milk-curdling enzymes.

Example I
Construction of the replicon screening vector pNZ10.

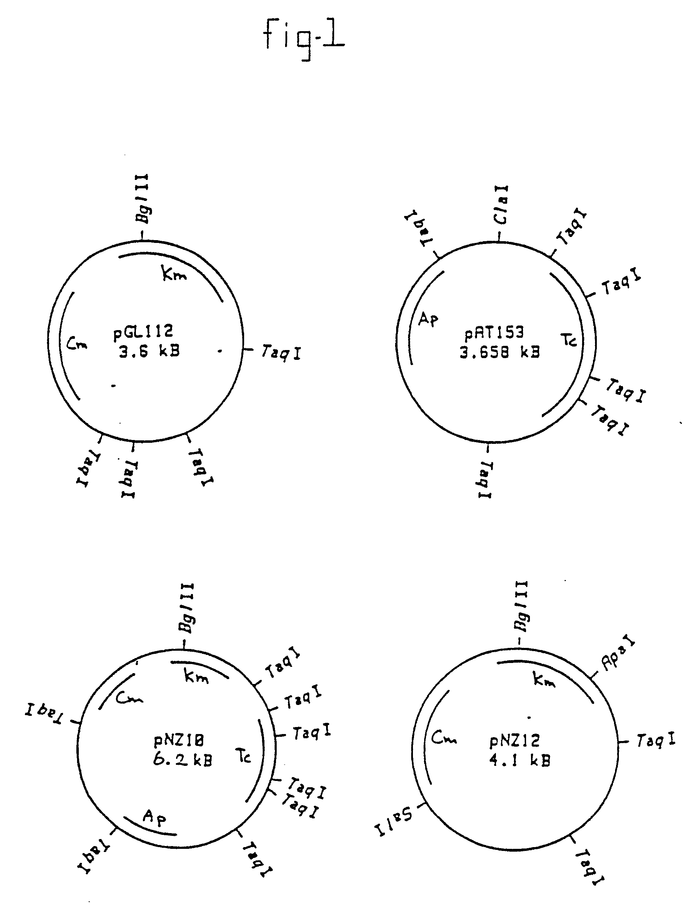

To isolate and identify DNA fragments which have replicon activity in lactic acid bacteria, a replicon screening vector termed pNZ10 was constructed. E. coli plasmid pAT153 (Nature 283 (1980) 216-218) and B. subtilis plasmid pGL112 (Mol. Gen. Genet. 190 (1983) 56-62) were used as the starting point for this purpose. A physical map of both plasmids is shown in Figure 1. The plasmid pGL112 consists of three small Taq I fragments which originate from pUB110 (J. Bacteriol. 134 (1978) 318-329) and which contain the replication signals of said plasmid (Plasmid 6 (1981) 67-77; J. Bacteriol. 154 (1983) 1184-1194) and a Taq I fragment having a size of 2.5 kb. This last named fragment contains the Taq I-Hpa II fragment with a size of 1.3 kb from pUB110 which contains the kanamycin (Km)-resistance gene (J. Bacteriol. 160 (1984) 413-420) and the Hpa II-Taq I fragment having a size of 1.2 kb from pC194 which contains the chloramphenicol (Cm)-resitance gene (J. Bacteriol. 150 (1982) 815-825). These two antibiotic-resistance markers are functional not only in Gram-positive bacteria such as S. aureus and B. subtilis , but also in E. coli (Proc. Natl. Acad. Sci. 75 (1978) 1433-1436; Mol. Gen. Genet. 183 (1981) 220-226).

To construct pNZ10, 5 $\mu$g of pGL112 DNA were isolated as described (Mol. Gen. Genet. 181 (1981) 424-433) from B. subtilis strain IG33, completely digested with units of the restriction enzyme Taq I (Boehringer, Mannheim) in 50 $\mu$l of a solution containing 10 mM tris.HCl (pH 7.5), 10 mM MgCl$_2$ and 10 mM 2-mercaptoethanol at 65°C for 2 hours. In addition, 5 $\mu$g of pAT153 DNA, isolated as described (Molecular Cloning: A Laboratory Manual, Cold Spring Harbor, 1982) from E. coli strain HB101, were linearized with 20 units of the restriction enzyme Cla I (Boehringer, Mannheim) in 50 $\mu$l of a solution containing 10 mM tris.HCl (pH 8.0) and 10 mM MgCl$_2$ at 37°C for 2 hours. The restriction enzyme fragments were then separated by electrophoresis in a 0.7% agarose gel (Appl. Environ. Microbiol. 43 (1982) 1272-1277) and the pAT153 DNA linearized with Cla I and also the Taq I fragment of pGL112 with a size of 2.5 kb were isolated as described (Anal. Biochem. 66 (1975) 213-220) from the gel. The isolated DNA fragments were mixed, precipitated with ethanol (Biochim. Biophys. Acta 607 (1980) 1-9) and then

6

dissolved in 17 μl of TE buffer (10 mM tris.HCl (pH 7.5); 1 mM EDTA), heated for 10 minutes at 68°C, and cooled to 4°C. Then 2 μl of 10-fold concentrated ligase buffer (500 mM tris.HCl (pH 7.5); 60 mM MgCl₂; 10 mM ATP; 50 mM dithiothreitol) and 1 unit of T4 DNA ligase (Boehringer, Mannheim) were added and the mixture was finally incubated at 14°C overnight. The restriction enzyme Cla I recognizes the sequence CTCGAG and generates protruding CG 5' ends: the restriction enzyme Taq I recognizes the sequence TCGA and also generates protruding CG 5' ends, so that Cla I and Taq I fragments can be ligated in a simple manner ("sticky end" ligation). Moreover Cla -Taq fusions can always be cut again by the restriction enzyme Taq I but they may not be recognized by the restriction enzyme Cla I. Half of the ligation mixture was used to transform E. coli strain BHB2600 as described (Molecular Cloning: A Laboratory Manual, Cold Spring Harbor). Transformants resistant to ampicillin (50 μg/ml) and chloramphenicol (10 μg/ml) were isolated with high frequency (2.1 × 10³ per μg of DNA). From some of these transformants the plasmid DNA was isolated on a micro scale (Nucl. Acids. Res. 7 (1979) 1513-1523) and analysed by means of, inter alia, Taq I digestions. One of the transformants contained a plasmid called pNZ10 with a structure as shown in Figure 1. Said plasmid pNZ10 replicates in E. coli by means of the pAT153 replicon and appears to bring about resistance to kanamycin (10 μg/ml) in said host. The pAT153 replicon is not, however, functional in B. subtilis nor in lactic acid bacteria such as S. lactis , as a result of which pNZ10 does not replicate in said bacteria and is therefore extremely suitable as a replicon screening vector. In addition, the pAT153 section of pNZ10 contains a large number of Taq I-recognition sites so that Taq I digestion of pNZ10 DNA leaves intact only the 2.5 kb fragment on which the antibiotic-resistance genes are located as shown in Figure 1.

Example II
Use of the replicon screening vector pNZ10 in the construction of the cloning vector pNZ12.

The usability of the replicon screening, vector can be checked in experiments in which pNZ10 DNA digested with the restriction enzyme Taq I, is ligated with restriction fragments which contain protruding CG 5' ends (as formed ih digestion with the restriction enzymes Cla I, Taq I, Asu II, Acy I, Mae II, Msp I, Nar I and also their isoschizomers, and in some cases Acc I; see Gene 33 (1985) 1-102) originating from, preferably, plasmid DNA of lactic acid bacteria. The ligation mixture can be used to transform B. subtilis protoplasts and to select Cm- and/or Km-resistant transformants. These transformants then contain recombinant plasmids which consist at least of the 2.5 kb pNZ10 Taq I fragment on which the antibiotic-resistance genes are located and also DNA fragments with replicon activity in B. subtilis . Since such fragments are not present in pNZ10 (see Example I) selection occurs for DNA fragments with a replicon activity originating from lactic acid bacteria. It is of course possible to isolate DNA fragments containing replicon activity and originating from other bacteria by replacing the lactic acid bacterium DNA in said procedure by DNA from the desired bacterium.

One of the vectors which has been constructed in the manner described above is the cloning vector pNZ12. For said construction plasmid DNA was completely isolated from the lactic acid bacterium strain S. lactis NCD0712 (J. Bacteriol. 154 (1983) 1-9) by a modification of the alkaline extraction method (Nucl. Acids Res. 7 (1979) 1513-1523; Third European Congress on Biotechnology, published by Verlag Chemie, III, 201-206). For this purpose the lactic acid bacteria were cultivated in 1 litre of glucose-M17 medium (Appl. Microbiol. 29 (1975) 807-813) to an E₆₀₀ of approximately 1.0 and harvested by centrifuging (10 minutes at 6,000 rpm; GSA rotor Sorvall) at 4°C, washed with sucrose buffer (25% sucrose; 20 mM tris.HCl (pH 8.0); 100 mM NaCl; 5 mM EDTA) and then suspended again in 75 ml sucrose buffer containing 500 μg of lysozyme (55700 u/ml; Sigma) per ml. This mixture was heated at 37°C until a clarification indicating complete "lysis" was observed (generally after 10 to 15 min. of incubation at 37°C) in micro-scale test experiments in which 100 μl of mixture was mixed with 200 μl of hydroxide solution (0.2N NaOH; 1% sodium dodecyl sulphate). At that instant 150 ml of freshly prepared hydroxide solution were added to 75 ml of mixture and the entire amount was kept on ice for 10 minutes after carefully mixing. Then 115 ml of ice-cold 3 M potassium acetate solution (pH 4.5 containing glacial acetic acid) were added; after mixing, the whole amount was kept on ice again for 10 min. After centrifuging (20 min.) at 4°C, the supernatant was filtered through cheesecloth in order to remove pellet particles which had been stirred up. The nucleic acids were precipitated from said supernatant with ethanol or isopropanol and subjected to CsCl-ethidium bromide equilibrium centrifugation as described (Appl. Environ. Microbiol. 43 (1982) 1272-1277) in order to isolate the plasmid DNA. Five μg of the S. lactis NCD0712 plasmid DNA thus isolated were again digested with the restriction enzyme Taq I and mixed with 2 μg of pNZ10 DNA also digested with the restriction enzyme Taq I . The restriction enzyme was removed by a phenol extraction followed by a chloroform extraction, and the DNA was precipitated with ethanol and then ligated in a final volume of 100 μl as

described. Half of this ligation mixture was used to transform B. subtilis strain IG33 protoplasts as described (Mol. Gen. Genet. 168 (1979) 111-115; Mol. Gen. Genet. 182 (1981) 39-43) and Cm$^R$ transformants were selected. One of said transformants contained a small (4.1 kb) plasmid which also produced Km$^R$ in B. subtilis as well as Cm$^R$. The physical map of said plasmid pNZ12 is also shown in Figure 1 and reveals that there are various unique restriction enzyme recognition sites in pN212, inter alia for the restriction enzymes Bgl II and Apa I (both located in the Km$^R$ gene) and Sal I. By means of hybridization experiments (Molecular Cloning; A Laboratory Manual, Cold Spring Harbor, 1982) it was possible to establish that pNZ12, in addition to the 2.5 kb Taq I fragment of pNZ10, contains two smaller Taq I fragments (1079 and 599 bp) which originate from the smallest S. lactis NCD0712 plasmid, pSH71 (J. Bacteriol. 154 (1983) 1-9). The full nucleotide sequence of said plasmid with a size of 2,060 bp has been determined by standard sequencing methods (Proc. Natl. Acad. Sci. 74 (1977) 5463-5467; Methods in Enzymology 101 (1983) 20-78; ibid. 65 (1980) 499-560) and is shown in Figure 2 together with a physical map of pSH71. The nucleotide sequence of the DNA with replicon activity present in pNZ12, which embraces the pSH71 DNA from position 2-1680, can be derived from this.

Those skilled in the art will realize that it is also possible to construct plasmids consisting of pNZ12 DNA, extended with one or more of the other five Taq I fragments of pSH71 which replicate in the same hosts as pNZ12. Moreover it has emerged that so-called mini-plasmids consisting of the 2.5 kb Taq I fragment of pNZ12 and the 599 bp Taq I fragment of pSH71 are also capable of replicating in B. subtilis and E. coli if the 2-1680 sequence of pSH71 is present on another replicon.

Example III
Determination of the host range and of the copy number of plasmid pNZ12.

In order to investigate the hosts in which pNZ12 can be used as a vector, pNZ12 DNA was transformed into representatives of both Gram-positive and Gram-negative bacteria of which it was expected that the antibiotic-resistance genes would come to expression therein. For this purpose E. coli strain MC1061 (J. Bacteriol. 143 (1983) 971-980) was transformed with 0.5 μg of pNZ12 DNA which resulted in approximately 10$^6$ transformants resistant to 10 μg of Cm/ml. Each of the twenty transformants investigated more closely for plasmid content contained a plasmid with the physical structure of pNZ12. Moreover, it emerged that pNZ12 also produced resistance to Km in E. coli (10 μg/ml). Protoplasts of S. aureus strain RN451 (J. Gen. Microbiol. 33 (1963) 121-136) were transformed as described (Mol. Gen. Genet. 168 (1979) 111-115; Proc. Natl. Acad. Sci. 79 (1982) 4108-4112) with 1 μg of pNZ12 DNA resulting in approximately 10$^3$ transformants resistant to 10 μg Cm/ml and 50 μg Km/ml. Some of these transformants were used to isolate plasmid DNA by the alkaline extraction method described above, starting from 1 ml of cell suspension and with an extra addition of 30 μg of lysostaphin per ml (220 μ/mg; Sigma) during the lysis step. In all the twelve transformants investigated there was plasmid DNA which had the physical structure of pNZ12.

S. lactis strain MG1363 (J. Bacteriol. 154 (1983) 1-9) was transformed by means of the protoplast-transformation system as follows: 50 ml of cells cultivated in glucose-M17 broth at 30° C to an E$_{600}$ of approximately 0.8 were harvested by centrifuging (10 minutes, 6,000 rpm; SS34 rotor Sorvall) at 20° C, and resuspended in 50 ml of GSM17 (M17 broth containing 0.5 M sucrose and I% glucose) which contained 40 mM ammonium acetate buffer (pH 7.0) and 1 mM magnesium acetate (FEMS Microbiol. Letters 9 (1980) 99-102) and also 1-4 mg of lysozyme per ml (55700 u/mg; Sigma) and then incubated for 2 hours at 37° C. The protoplasts were collected by centrifuging (10 minutes, 12,000 rpm; SS34 rotor Sorvall) at 20° C, washed with 20 ml of SMMC buffer (SMM buffer (Mol. Gen. Genet. 168 (1979) 111-115) with 10 mM CaCl$_2$) and resuspended in 5 ml of SMMC. The transformation of the washed protoplasts was carried out by adding to 100 μl of the suspension 10 μl of pNZ12 DNA which had been previously mixed with an equal volume of twice concentrated SMMC containing 1 μg of pNZ12 DNA, followed by addition of 300 μl of 40% polyethylene glycol 6000 in SMMC. After incubating for 5 minutes at 20° C, 1 ml of GSM17 was added, the protoplasts were collected by centrifuging (1 minute, 12,000 rpm in an Eppendorf 5414 S centrifuge) and dissolved in 1 ml of GSM17. After an expression period of 1 hour at 30° C various dilutions of the protoplasts prepared in GSM17 and solidified in 3 ml of GSM17 with 0.7% agar were plated out on GSM17 agar plates containing 1.5% agar and 4 μg Cm/ml.

After three days of incubation at 30° C, approximately 10$^3$ Cm$^R$ transformants were found per μg of pNZ12 DNA. Some of these transformants were used to isolate plasmid DNA by the alkaline extraction method described above, starting from 1 ml of cell suspension. In all the transformants investigated there was plasmid DNA which had the physical structure of pNZ12.

The copy number of pNZ12 in the hosts B. subtilis , E. coli and S. lactis was determined by a hybridization method in which all the DNA was liberated from a known quantity of logarithmically growing cells and transferred to Gene Screen Plus (New England Nuclear) filters as described (Nucl. Acids Res. 7 (1979) 1541-1550). Said filters were then hybridized with $^{32}$P-labelled pNZ12 DNA (J. Mol. Biol. 113 (1977) 237-251), washed, dried and autoradiographed (Molecular Cloning: A Laboratory Manual, Cold Spring Harbor 1982). The quantity of radioactivity (a measure of the quantity of pNZ12 DNA in the samples) was then determined and compared with the quantity of radioactivity found in a hybridization experiment carried out in parallel, in which known quantities of pure pNZ12 DNA were placed on the filters. Allowing for the number of bacteria in the initial culture, it was possible to derive the copy number of pNZ12 per bacterium cell from these data. From the results listed in Table 1 it emerges that pNZ12 has a copy number in E. coli and in S. lactis which is high and comparable with that in the cloning vector pAT153 (100 - 150 copies per cell) much used in E. coli . Moreover, it emerged from the determination of the plasmid DNA yield that pNZ12 also has such a high copy number in S. aureus . In B. subtilis , on the other hand, the copy number of pNZ12 is a factor of five lower.

### TABLE 1

| Host | | Copy number of pNZ12 per cell |
|------|---|---|
| E. coli | MC1061 | 98 |
| B. subtilis | IG33 | 19 |
| S. lactis | MG1363 | 110 |

Example IV
Cloning of the S. lactis phospho-β-D-galactosidase gene in the vector plasmid pNZ12 and expression in E. coli , B. subtilis and S. lactis .

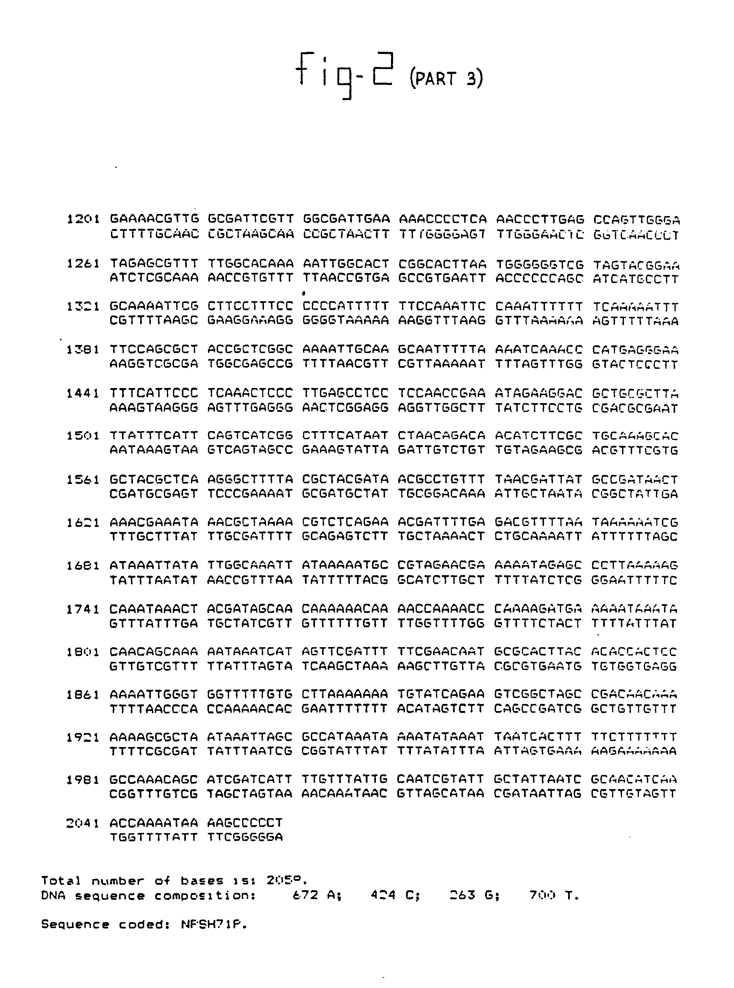

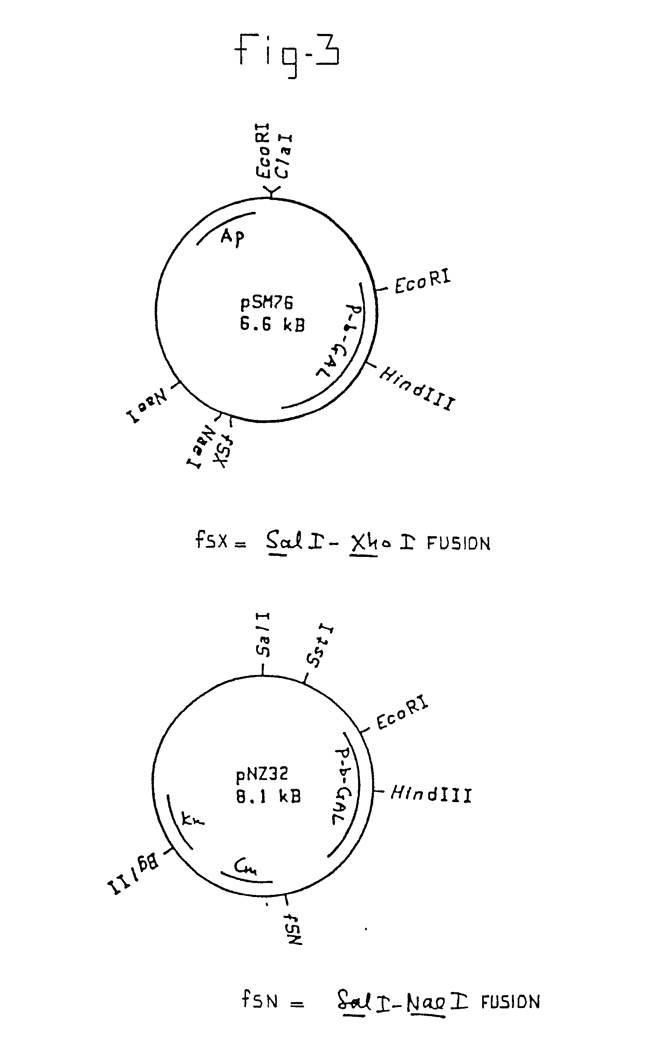

To check the usability of the cloning vector pNZ12 described above, the phospho-β-D-galactosidase (P-β-Gal) gene of S. lactis was cloned in S. lactis , use being made of intermediary hosts in which pNZ12 can replicate. The P-β-Gal gene was chosen because the gene product can be determined spectrophotometrically in a simple manner by means of the chromogenic substrate o-nitrophenyl-β-D-galactopyranoside-6-phosphate (Agric. Biol. Chem. 43 (1979) 2389-2390) and is therefore very usable as a model gene for measuring gene expression. The starting point was the plasmid pSM76, which consists of pAT153 in which the Cla I-Sal I fragment is replaced by a 3.5 kb Cla I-Xho I fragment from the S. lactis NCD0712 plasmid pLP712 which contains the P-β-Gal gene (Genetic Engineering of Microorganisms Important for Agro-Food Industries, CEC, Marseille (1984) 34-35. Because the Sal I-Xho I fusion is not recognized by Sal I or Xho I, the P-β-Gal gene was liberated from pSM76 by digestion with Cla I and Nae I. It had been found previously that Nae I does not cut the S. lactis section of pSM76 while a Nae I recognition site is located 120 bp from the Cla I-Xho I fusion in the pAT153 section (Cold Spring Harbor Symp. Quant. Biol. 43 (1978) 77-85). Because, furthermore, the restriction enzyme Nae I generates DNA fragments with so-called blunt ends, a blunt-end ligation was chosen in order to insert the DNA fragment which contains the P-β-Gal gene into pNZ12; see Figure 3, in which fSN indicates the Sal I-Nae I fusion. For this purpose 5 μg of pSM76 DNA were digested with Cla I as described, the linear DNA was precipitated with ethanol and taken up in 50 μl of polymerase buffer (25 mM tris.HCl, pH 7.8; 10 mM MgCl₂; 10 mM dithiothreitol; 0.1 mM dATP; 0.1 mM dGTP; 0.1 mM dCTP; 0.1 mM TTP). The Cla I recognition site was then filled in by adding 5 units of DNA polymerase I large (Klenow) fragment (Boehringer, Mannheim) and incubating for 20 minutes at 20° C. The reaction was stopped by incubating for 10 minutes at 65° C and the DNA was precipitated with ethanol, then taken up in 50 μl of Nae I buffer (10 mM tris.HCl, pH 7.5; 10 mM MgCl₂; 50 mM NaCl; 10 mM 2-mercaptoethanol) and digested with 20 units of the restriction enzyme Nae I (Boehringer, Mannheim) for 2 hours at 37° C. The 3.9 kb fragment which contains the P-β-Gal gene was then separated from the pAT153 fragments by means of agarose-gel electrophoresis, isolated, precipitated with ethanol, and taken up in 20 μl of TE buffer (see Example I). The vector pNZ12 was linearized and provided with blunt ends as follows: 5 μg of pNZ12 were digested with 20 units of the restriction enzyme Sal I (Boehringer, Mannheim) in 50 μl of Sal I buffer (50 mM tris.HCl, pH 7.5; 100 mM NaCl; 10 mM MgCl₂; 10 mM 2-mercaptoethanol) for 2 hours at 37°C. The linearized pNZ12 DNA was then precipitated with ethanol and taken up in 50 μl of polymerase buffer. The Sal I ends were filled in with DNA polymerase I large (Klenow) fragment as described, and the DNA was separated by means of agarose-gel electrophoresis, isolated, precipitated with ethanol and taken up in 20 μl of TE buffer. Ten μl of the filled-in Cla I-Nae I fragment described above which contains the P-β-Gal gene and 10 μl of the vector pNZ12, linearized with Sal I and also filled in, were combined and, after adding 2.5 μl of 10-fold concentrated ligase buffer, were ligated with 2.5 units of T4 DNA polymerase (see Example I) for 20 hours at 15°C. Ten μl of said ligation mixture was used to transform E. coli strain MC1061 to $Cm^R Km^R$. Transformants containing the P-β-Gal gene were identified by means of colony hybridization (Proc. Natl. Acad. Sci. 72 (1975) 3961-3966) with $^{32}P$-labelled pSM76 DNA. One of the positive clones contained a plasmid pNZ32 with the structure shown in Figure 3. After treatment with toluene, cells which contained pNZ32 exhibited P-β-Gal activity measured as explained earlier, which indicates that the intact S. lactis P-β-Gal gene was cloned in pNZ12. In order to demonstrate the usability of pNZ12 as a vector for P-β-Gal-gene when cloning in B. subtilis and in S. lactis , pNZ32 DNA was isolated from E. coli strain MC1061 and used to transform protoplasts of B. subtilis and of S. lactis as described in Example II. In B. subtilis strain IG33 approximately $10^5$ $Cm^R Km^R$ transformants were found per μg; from some of these plasmid DNA was isolated, which was identical to pNZ32 as regards physical organization.

In S. lactis strain MG1363 approximately $10^3$ $Cm^R$ transformants were found per μg; here too the physical organization of the plasmid DNA of some of these transformants proved to be identical to that of pNZ32. In both Gram-positive bacteria, as well as in E. coli , the P-β-Gal gene is expressed (see Table 2). This indicates that pNZ12 is a usable cloning vector for said hosts. Moreover, this means that the P-β-Gal gene is a usable model gene both for E. coli and B. subtilis and for lactic acid bacteria.

### TABLE 2

### P-β-Gal activity

### (nmol/min/mg of protein)

|  |  | without pNZ32 | with pNZ32 |
|---|---|---|---|
| E. coli | MC1061 | 1 | 186 |
| B. subtilis | IG33 | 0.1 | 10 |
| S. lactis | MG1363 | 1 | 58 |

Example V
Construction of the promoter screening vector pNZ220.

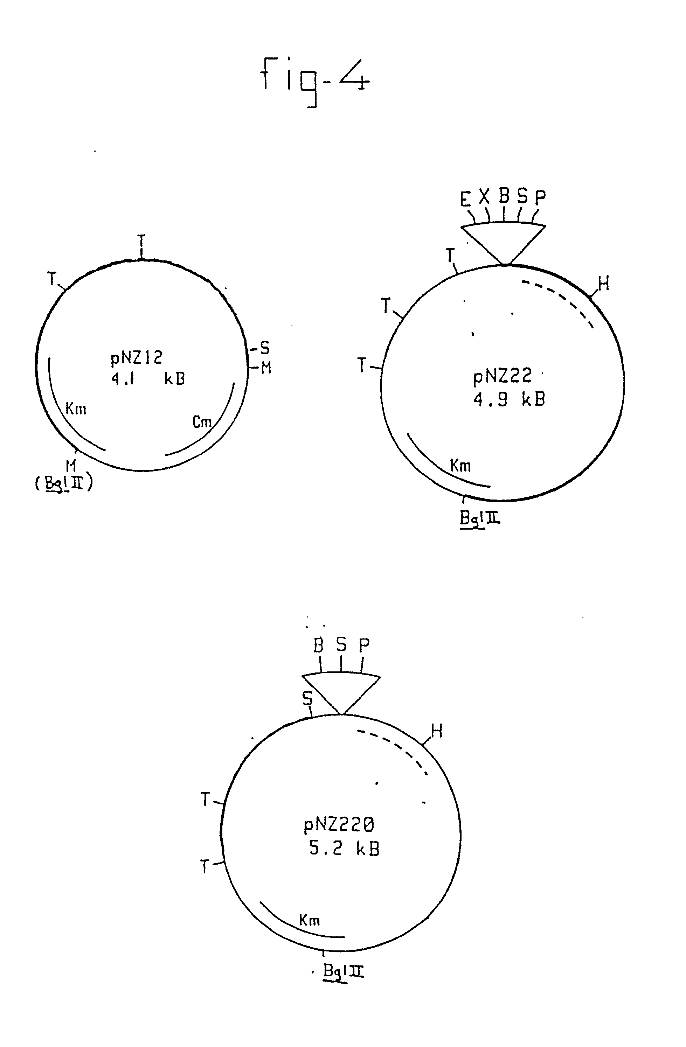

The B. subtilis plasmid pPL603 is a so-called promoter screening vector which can be used to isolate DNA fragments with promoter activity (J. Bacteriol. 148 (1981) 1162-1165). For this purpose, in addition to the replication functions and the $Km^R$ gene of pUB110 (see Example I), said plasmid also contains the structural gene which codes for the enzyme chloramphenicol acetyl-transferase (CAT) originating from B. pumilis which is deprived of the CAT promoter active in vegetative cells. Immediately upstream of the CAT gene there is located an Eco RI-Pst I fragment with a size of 203 bp, which can be replaced in a simple manner by the Eco RI-Pst I polylinker sequence as has been described for the construction of pPL703 for which the 21 bp polylinker from M13mp7 was used (J. Bacteriol. 155 (1983) 1399-1406). As a result of this not only extra, unique restriction enzyme recognition sites are created immediately upstream of the structural CAT gene but, in addition, said manipulation completely deprives the CAT gene of promoter sequences which are functional in B. subtilis (J. Bacteriol. 158 (1984) 784-790). In an analogous manner the pPL603 Eco RI-Pst I fragment is replaced in the plasmid pNZ22 used here (Figure 4) by the 28 bp Eco -Pst polylinker from M13mp8 (Gene 19 (1984) 269-276), as a result of which unique restriction enzyme recognition sites for Sma I, Bam HI and Sal I are added. In Figure 4 the recognition sites for the restriction enzymes Eco RI, Xma I, Bam HI, Sal I, Pst I, Hind III, Taq I, and Mbo I are indicated respectively by the letters E, X, B, S, P, H, T, and M. Mbo I is an isoschizomer of Sau 3A, and Xma I of Sma I. The location of the antibiotic resistance genes is indicated, the promoterless CAT gene of B. pumilis being indicated by a broken line. The emboldened lines in pNZ12 and pNZ22 indicate those sections which are combined in pNZ220.

The usability of a promoter screening vector such as pNZ22 can be considerably increased by replacing the pUB110 replication functions by those from pNZ12 (see Examples II and III), as a result of which the host range is expanded. Because of the favourable location of the Sau 3A restriction enzyme recognition sites and the presence of the same Km$^R$ gene (the kanamycin nucleotidyltransferase KNT gene from pUB110, J. Bacteriol. 160 (1984) 413-420) in pNZ22 and pNZ12, this can be done in a simple manner by replacing the 2.2 kb Bam HI-Bgl II fragment of pNZ22 by the 3.5 kb Sau 3A fragment of pNZ12 (see Fig. 4). Because, moreover, the Sau 3A, Bgl II and Bam HI restriction fragments contain the same 5' GATC protruding ends, sticky end ligation is possible. In addition, in said construction, the Bam HI recognition site is reformed again by the sequence GATCC.... in the small Sau 3A-Sal I fragment of pNZ12 (originating from pC194: J. Bacteriol. 150 (1982) 815-825). An example of such a plasmid is the promoter screening vector pNZ220. The starting point for the construction thereof was 5 µg of pNZ22 DNA which were digested with 20 units of the restriction enzymes Bam HI and Bgl II (Boehringer, Mannheim) in 50 µl of Sal I buffer (Example IV) for 2 hours at 37° C. The restriction fragments were separated by means of agarose-gel electrophoresis, and the Bam HI-Bgl II fragment with a size of 2.2 kb was isolated and concentrated as described above and dissolved in 20 µl of TE buffer. Five µg of pNZ12 DNA were digested in 50 µl of Sal I buffer with 10 units of the restriction enzyme Sau 3A (Boehringer, Mannheim) for 2 hours at 37° C, and the 3.5 kb Sau 3A fragment was isolated and concentrated as described after separation, and dissolved in 20 µl of TE buffer. Ten µl of each of the two fragments, the Bam HI-Bgl II fragment of pNZ22 and the Sau 3A fragment of pNZ12, were mixed and ligated in a volume of 50 µl by means of T4 DNA ligase as described. This ligation mixture was used to transform E. coli strain MC1061 as described, and transformants which are resistant to 6 µg Km/ml were selected with a frequency of approximately 10² per µg of DNA. In one of these transformants there was a plasmid, pNZ220, with the structure shown in Figure 4. Said plasmid contains the replication function of pNZ12 which makes replication in B.subtilis , E.coli , S.aureus and lactic acid bacteria possible.

By now linearizing pNZ22 or pNZ220 with one of the restriction enzymes which recognize recognition sites situated upstream of the CAT gene and ligating DNA fragments of various origins, but preferably originating from lactic acid bacteria, to the linearized plasmid DNA, recombinant plasmids can be constructed which can be transformed into B.subtilis , and in the case of pNZ220 additionally into E.coli , S.aureus and lactic acid bacteria. Transformants which are resistant to Cm can then have DNA fragments with promoter activity, the level of Cm resistance being a measure of the strength of the promoter.

## Example VI
Construction of the expression vector pNZ221.

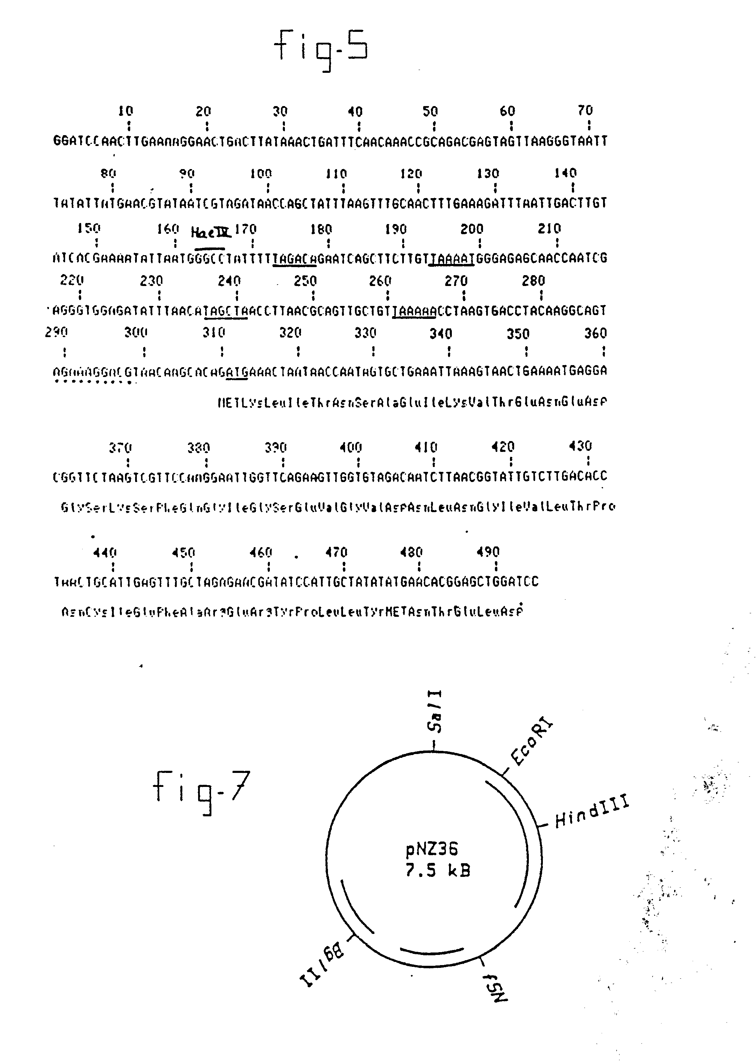

The usability of the promoter screening vectors pNZ22 and pNZ220 for isolating DNA fragments with promoter activity was checked in the construction of the expression vector pNZ221, consisting of pNZ220 containing a Sau 3A fragment on which a strong lactic-acid-specific promoter is located. The virulent S.cremoris bacteriophage 0 SK11G (FEMS Microbiol. Letters 23 (1984) 175-178) was chosen as a source of DNA with promoter activity. It is known that bacteriophages generally have very powerful promoters (Gene 15 (1981) 81-93; Proc.Natl.Acad.Sci. 78 (1981) 4936-4940; J.Mol.Biol. 153 (1981) 527-544). ØSK11G bacteriophage was multiplied on S.cremoris strain SK112 and purified as described (FEMS Microbiol.Letters 23 (1984) 175-178). From approximately 10¹³ ØSK11G particles approximately 500 µg of ØSK11G DNA were isolated as described for bacteriophage lambda (Molecular Cloning: A Laboratory Manual, Cold Spring Harbor 1982). The ØSK11G DNA is a linear double-stranded DNA with a size of approximately 50 kb and contains cohesive ends. The ØSK11G DNA was dissolved in TE buffer to a concentration of 500 µg/ml. Ten µl of said ØSK11G DNA were completely digested with the restriction enzyme Sau 3A under conditions described in Example V. The restriction enzyme was removed by a phenol extraction and a chloroform extraction, and the DNA fragments were precipitated with ethanol and dissolved in 20 µl of TE buffer. Then 5 µg of pNZ22 DNA were linearized with the restriction enzyme Bam HI as described above (Example V), deproteinized, precipitated, and dissolved in 50 µl of TE buffer. Ten µl of this linear pNZ22 DNA were mixed with 20 µl of the ØSK11G-Sau 3A fragments described above, and the DNA mixture was ligated with T4 DNA ligase in a volume of 50 µl. Half of this ligation mixture was used to transform protoplasts of B.subtilis strain IG33, and transformants which were resistant to 10 µg Cm/ml were selected; approximately 10³ of such transformants were found. One of the said transformants proved resistant to approximately 75 µg Cm/ml, and the plasmid DNA in this transformant was investigated more closely. Said plasmid, pNZ250, contained a Sau 3A fragment with a size of 495 bp, originating from ØSK11G as it was possible to establish by means of hybridization experiments, in the Bam HI recognition site of pNZ22. The nucleotide sequence

11

of said 495 bp fragment having promoter activity has been determined by means of standard sequencing methods (see Example II) and is shown in Figure 5. In said figure two promoter sequences are underlined and an RBS is shown by a dotted line; in addition, an open reading frame is shown which starts with an ATG initiation codon and with translation into amino acids. From the DNA sequence around the Sau 3A recognition sites it may be deduced that said sites in pNZ250 are also recognized by the restriction enzyme Bam HI. As a result of this it is possible to liberate the 495 bp promoter fragment in a simple manner from pNZ250 by digestion with the restriction enzyme Bam HI. Use was made of this in subcloning the 495 bp promoter fragment in pNZ220. For this purpose 5 μg of pNZ250 DNA were digested with the restriction enzyme Bam HI; the promoter fragment was isolated after polyacrylamide gel electrophoresis (Molecular Cloning: A Laboratory Manual, Cold Spring Harbor 1982), concentrated and dissolved in 10 μl of TE buffer. Then 5 μg of pNZ220 DNA were linearized with the restriction enzyme Bam HI, deproteinized, concentrated and taken up in 50 μl of TE buffer. Ten μl of each of the fragments were ligated by means of T4 DNA ligase in a volume of 50 μl, and the ligation mixture was used to transform B.subtilis 1G33 protoplasts and Cm$^R$ transformants were analysed for their plasmid content. In each of the transformants investigated there was a plasmid with a structure like that of pNZ221 (Figure 6). Because the replication functions of pNZ12 (see Examples II and III) are present in pNZ221, this expression plasmid can be used not only in B.subtilis but also in E.coli , S.aureus and lactic acid bacteria.

The location and the orientation of the DNA sequences with promoter activity in the 495 bp Bam HI fragment of pNZ221 were investigated by means of subcloning experiments, use being made, inter alia, of the asymmetric Hae III recognition site (see Figure 5, position 163). Only the righthand Hae III-Bam HI fragment proved to have promoter activity in both B.subtilis and E.coli . In the DNA sequence of this fragment section (see Figure 5) two regions appear to occur which exhibit homology with the promoter consensus DNA sequence which has been drawn up for E.coli and B.subtilis promoters (Mol.Gen.Genet. 186 (1982) 339-346). These two inferred S.cremoris ØSK11G promoters are followed by the sequence AGAAAGGACG (see Figure 5), which exhibits strong complementarity with the 3' ends of the 16S rRNA of the Gram-positive bacteria B.subtilis , S.aureus and S.lactis (J.Biol.Chem. 256 (1981) 11283-11292; J.Gen.Microbiol. 131 (1985) 543-551), and can therefore function as so-called ribosome binding site. The fact that there is located at eleven bp from said RBS a start codon (ATG) which is followed by a so-called open reading frame which comprises 61 codons up to the Bam HI recognition site, suggests the functionality of said RBS.

The functionality of the inferred promoter sequences and of the said presumed RBS having the ATG start codon in bringing genes without these signals to expression is demonstrated in the construction of plasmid pNZ280 in E.coli . This plasmid consists of plasmid pMC1403 (J.Bacteriol. 143 (1980) 971-980) in which the 495 bp promoter fragment has been introduced into the unique Bam HI recognition site. The β-galactosidase (β-Gal) gene does not come to expression in pMC1403 because both the promoter and RBS of the β-Gal gene in pMC1403 are missing; the first seven amino terminal amino acids are also missing.

Expression of the β-Gal gene can only be brought about if the inserted fragment has promoter activity, an RBS with ATG start codon and a reading frame which is in phase with the eighth codon of the β-Gal segment.

Five μg of pMC1403 DNA were digested with 20 units of the restriction enzyme Bam HI, treated with phenol, concentrated and taken up in 50 μl of TE buffer. Purified 495 bp promoter fragment (for which the starting point was 5 μg of pNZ250 DNA as described earlier) was ligated with 5 μl of linearized pMC1403 DNA by means of T4 DNA ligase. Competent cells of E.coli strain MC1061 were transformed with the ligation mixture, and plated out on LB plates which contained 100 μg of carbenicillin and 40 μg of 5-bromo-4-chloro-3-indolyl-β-D-galactoside per ml. In addition to a large number of colourless transformants, blue-coloured transformants also proved to be present; blue coloration indicates β-galactosidase activity (Experiments in Molecular Genetics, Cold Spring Harbor 1972). In all the blue-coloured transformants investigated the 495 bp promoter fragment appeared to be inserted in the unique Bam HI recognition site of pMC1403, while the plasmid DNA of the colourless transformants corresponded to the structure of pMC1403. Finally, the functionality of the 495 bp Bam HI promoter fragment in lactic acid bacteria was demonstrated by isolating said fragment as a 0.6 kb Sal I fragment from pNZ221 (Figure 6) and inserting it in the unique Sal I recognition site which is upstream of the P-β-Gal gene in pNZ36 (Figure 7). The plasmid pNZ36 is derived from pNZ32 (see Example IV) and is constructed by removing the approximately 0.6 kb Sal I-Sst I fragment from pNZ32 and treating the residual fragment with DNA polymerase I (Klenow fragment) and T4 DNA polymerase (Molecular Cloning: A Laboratory Manual, Cold Spring Harbor 1982), as a result of which a SalI recognition site is again reformed after circularization by means of T4 DNA ligase.

The promoter fragment is cloned in two orientations in said Sal I recognition site: the promoter sequence in the same direction as the P-β-Gal gene resulting in pNZ367, and the promoter sequence in the opposite direction resulting in pNZ361. Both plasmids have been transformed into S.lactis and the expression of the P-β-Gal gene has been measured in both cases (Table 3). It emerges that high P-β-Gal activity is measured only if the promoter fragment is located in the correct orientation for the P-β-Gal gene, which indicates that said promoter fragment functions efficiently in lactic acid bacteria.

**TABLE 3**

**P-β-Gal activity**

**(nmol/min/mg of protein)**

**in S.lactis MG1363**

| | |
|---|---|
| pNZ361 | 20 |
| pNZ367 | 78 |

Legend

Figure 1 :

Physical maps of the plasmids pGL112, pAT153, pNZ10 and pNZ12 showing the location of the antibiotic-resistance genes.

Figure 2 :

Physical map of the S.lactis plasmid pSH71 and the nucleotide sequence of said plasmid.

Figure 3 :

Physical map of the plasmids pSM76 and pNZ32 showing the location of the antibiotic-resistance genes and the P-β-Gal gene.

Figure 4 :

Physical map of the plasmids pNZ22, pNZ12 and pNZ220. The recognition sites for the restriction enzymes Eco RI, Xma I, Bam HI, Sal I, Pst I, Hind III, Taq I, Mbo I are indicated by the letters E, X, B, S, P, H, T and M respectively. Mbo I is an isoschizomer of Sau 3A and Xma I of Sma I. The location of the antibiotic-resistance genes is indicated, the promoterless CAT gene of B.pumilis being shown by a broken line. The emboldened lines in pNZ12 and pNZ22 indicate the sections which are combined in pNZ220.

Figure 5 :

Nucleotide sequence of the 495 bp Sau 3A fragment of pNZ250 containing two promoter sequences (underlined), an RBS (dotted line) and an open reading frame starting with an ATG initiation codon the translation of which into amino acids is given.

Figure 6 :

Physical map of the expression vector pNZ221 showing the location of the antibiotic-resistance genes; the promoterless CAT gene of B.pumilis is shown by a broken line.

Figure 7 :

Physical map of the plasmid pNZ36 showing the location of the antibiotic-resistance genes and also the location and orientation of the P-β-Gal gene.

**Claims**

1. DNA fragment coding for (a) a replicon activity in lactic acid bacteria and (b) at least one antibiotic-resistance gene, characterized in that the DNA fragment which codes the replicon activity has the DNA sequence extending from base pair 2 to base pair 1680 of the sequence shown in Figure 2, parts 2 and 3 or a mutated form thereof having the same activity.

2. DNA fragment according to claim 1, characterized in that it also has a DNA sequence which codes for a promoter activity and a ribosome binding side in lactic acid bacteria as shown in Figure 5 or a mutated form thereof having the same activity.

3. Method for isolating DNA fragments which code for replicon activity, characterized in that the replicon screening vector pNZ10 shown in Figure 1 is used in those micro-organisms in which at least one antibiotic-resistance gene is expressed, but in which the E.coli replicon is not functional.

4. Method for isolating DNA fragments which code for promoter activity, characterized in that a promoter screening vector pNZ22 or pNZ220 as shown in Figure 4 is used in lactic acid bacteria.

5. Recombinant DNA plasmid comprising (a) a DNA fragment which codes for a replicon activity in lactic acid bacteria and (b) at least one antibiotic-resistance gene, characterized in that the DNA fragment which codes for a replicon activity has the DNA sequence extending from base pair 2 to base pair 1680 of the sequence shown in Figure 2, parts 2 and 3 or a mutated form thereof having the same activity.

6. Recombinant DNA plasmid according to claim 5, characterized in that the DNA fragment which codes for the promoter activity and the ribosome binding site in lactic acid bacteria has the DNA sequence as shown in Figure 5 or a mutated form thereof having the same activity.

7. Recombinant DNA plasmid according to claim 5 or 6, characterized in that it also contains a gene which codes for a specific protein.

8. Recombinant DNA plasmid according to claim 7, characterized in that the gene is the phospho-β-galactosidase gene or a gene which codes for chymosin or another caseolytic enzyme or a precursor thereof.

9. Host organism containing one or more recombinant DNA plasmids, characterized in that the recombinant DNA plasmids correspond to the plasmids defined in claims 5-8.

10. Host organism according to claim 9, characterized in that the host organism comprises lactic acid bacteria, Streptococcus aureus, Bacillus subtilis or Escherichia coli .

11. Host organism according to claim 10, characterized in that the host organism comprises lactic acid bacteria.

12. Host organism according to claim 11, characterized in that the host organism is Streptococcus lactis .

13. Method for the preparation of proteins by cultivating a micro-organism containing one or more recombinant DNA plasmids, characterized in that a microorganism as defined in one or more of the claims 9-12 is cultivated.

**Revendications**

14

1. Fragment d'ADN codant pour (a) une activité de réplicon dans les bactéries lactiques et (b) au moins un gène de résistance aux antibiotiques, caractérisé en ce que le fragment d'ADN qui code l'activité de réplicon a la séquence d'ADN s'étendant de la paire de base 2 à la paire de base 1680 de la séquence montrée dans la figure 2, parties 2 et 3 ou une de ses formes mutées ayant la même activité.

2. Fragment d'ADN selon la revendication 1, caractérisé en ce qu'il a également une séquence d'ADN qui code pour une activité de promoteur et un site de liaison ribosomique dans les bactéries lactiques comme on le voit dans la figure 5 ou une de ses formes mutées ayant la même activité.

3. Procédé pour isoler des fragments d'ADN qui codent pour une activité de réplicon, caractérisé en ce que le vecteur de séleotion de réplicon pNZ10 présenté dans la figure 1 est utilisé dans les microorganismes dans lesquels au moins un gène de résistance aux antibiotiques est exprimé, mais dans lequel le réplicon d'E. coli n'est pas fonctionnel.

4. Procédé pour isoler des fragments d'ADN qui codent pour une activité de promoteur, caractérisé en ce qu'on utilise un vecteur de sélection de promoteur PNZ22 ou pNZ220 tel que présenté dans la figure 4 dans les bactéries lactiques.

5. Plasmide d'ADN recombinant comprenant (a) un fragment d'ADN qui code pour une activité de réplicon dans les bactéries lactiques et (b) au moins un gène de résistance aux antibiotiques, caractérisé en ce que le fragment d'ADN qui code pour une activité de réplicon a la séquence d'ADN s'étendant de la paire de base 2 à la paire de base 1680 de la séquence présentée dans la figure 2, parties 2 et 3, ou une de ses formes mutées ayant la même activité.

6. Plasmide d'ADN recombinant selon la revendication 5, caractérisé en ce que le fragment d'ADN qui code pour l'activité de promoteur et le site de liaison ribosomique dans les bactéries lactiques a la séquence d'ADN présentée dans la figure 5 ou une de ses formes mutées ayant la même activité.

7. Plasmide d'ADN recombinant selon la revendication 5 ou 6, caractérisé en ce qu'il contient également un gène qui code pour une protéine spécifique.

8. Plasmide d'ADN recombinant selon la revendication 7, caractérisé en ce que le gène est le gène de la phospho-β-galactosidase ou un gène qui code pour la chymosine ou une autre enzyme caséolytique ou un de leurs précurseurs.

9. Organisme hôte contenant un ou plusieurs plasmides d'ADN recombinant, caractérisé en ce que les plasmides d'ADN recombinant correspondent aux plasmides définis dans les revendicaticns 5-8.

10. Organisme hôte, selon la revendication 9, caractérisé en ce que l'organisme hôte comprend les bactéries lactiques, les Streptococcus aureus , les Bacillus subtilis ou les Escherichia coli .

11. Organisme hôte selon la revendication 10, caractérisé en ce que l'organisme hôte comprend des bactéries lactiques.

12. Organisme hôte selon la revendication 11, caractérisé en ce que l'organisme hôte est Streptococcus lactis.

13. Procédé de préparation de protéines par mise en culture d'un microorganisme contenant un ou plusieurs plasmides d'ADN recombinant, caractérisé en ce qu'on cultive un microorganisme tel que défini dans l'une ou plusieurs des revendications 9-12.

## Ansprüche

1. DNA-Fragment, das für (a) eine Replikonaktivität in Milchsäurebakterien und (b) mindestens ein Antibiotikaresistenzgen kodiert, dadurch **gekennzeichnet**, daß das DNA-Fragment, das die Replikonaktivität kodiert, die DNA-Sequenz, die sich von Basenpaar 2 bis Basenpaar 1680 der in Fig. 2, Teile 2 und 3 gezeigten Sequenz erstreckt, oder eine mutierte Form derselben mit derselben Aktivität, hat.

2. DNA-Fragment gemäß Anspruch 1, dadurch gekennzeichnet, daß es auch eine DNA-Sequenz, die für eine Promotoraktivität und eine Ribosomenbindungsstelle in Milchsäurebakterien kodiert, wie in Fig. 5 gezeigt, oder eine mutierte Form derselben mit derselben Aktivität, hat.

3. Verfahren zur Isolierung von DNA-Fragmenten, die für Replikonaktivität kodieren, dadurch gekennzeichnet, daß der in Fig. 1 gezeigte Replikon-Screen-Vektor pNZ10 in denjenigen Miroorganismen verwendet wird, in denen mindestens ein Antibiotikaresistenzgen exprimiert wird, aber in denen das E.coli-Replikon nicht funktionell ist.

4. Verfahren zur Isolierung von DNA-Fragmenten, die für Promotoraktivität kodieren, dadurch gekennzeichnet, daß ein Promotor-Screen-Vektor pN222 oder pNZ220, wie in Fig. 4 gezeigt, in Milchsäurebakterien verwendet wird.

5. Rekombinantes DNA-Plasmid, das (a) ein DNA-Fragment, das für eine Replikonaktivität in Milchsäurebakterien und (b) mindestens ein Antibiotikaresistenzgen kodiert, umfaßt, dadurch gekennzeichnet, daß das DNA-Fragment, welches für eine Replikonaktivität kodiert, die DNA-Sequenz, die sich von Basenpaar 2 bis Basenpaar 1680 der in Fig. 2, Teile 2 und 3 gezeigten Sequenz erstreckt, oder eine mutierte Form derselben mit derselben Aktivität, hat.

6. Rekombinates DNA-Plasmid gemäß Anspruch 5, dadurch gekennzeichnet, daß das DNA-Fragment, das für die Promotoraktivität und die Ribosomenbindungsstelle in Milchsäurebakterien kodiert, die DNA-Sequenz, wie sie in Fig. 5 gezeigt ist, oder eine mutierte Form derselben mit derselben Aktivität, hat.

7. Rekombinantes DNA-Plasmid gemäß Anspruch 5 oder 6, dadurch gekennzeichnet, daß es auch ein Gen enthält, welches für ein spezifisches Protein kodiert.

8. Rekombinantes DNA-Plasmid gemäß Anspruch 7, dadurch gekennzeichnet, daß das Gen das Phospho-β-galactosidase-Gen oder ein Gen ist, welches für Chymosin oder ein anderes caseolytisches Enzym oder einen Vorläufer desselben kodiert.

9. Wirtsorganismus, der ein oder mehrere DNA-Plasmide enthält, dadurch gekennzeichnet, daß die rekombinanten DNA-Plasmide den in den Ansprüchen 5 bis 8 definierten Plasmiden entsprechen.

10. Wirtsorganismus gemäß Anspruch 9, dadurch gekennzeichnet, daß der Wirtsorganismus Milchsäurebakterien, Streptococcus aureus, Bacillus subtilis oder Escherichia coli umfaßt.

11. Wirtsorganismus gemäß Anspruch 10, dadurch gekennzeichnet, daß der Wirtsorganismus Milchsäurebakterien umfaßt.

12. Wirtsorganismus gemäß Anspruch 11, dadurch gekennzeichnet, daß der Wirtsorganismus Streptococcus lactis ist.

13. Verfahren zur Herstellung von Proteinen durch Kultivierung eines Mikroorganismus, der ein oder mehrere rekombinante DNA-Plasmide enthält, dadurch gekennzeichnet, daß ein Mikroorganismus, wie er in einem oder mehreren der Ansprüche 9 bis 12 definiert ist, kultiviert wird.

# fig.1

EP 0 228 726 B1

Fig-2 (PART 1)

pSH71
2060 bp

Fig-6

pNZ221
5.7 kB

18

EP 0 228 726 B1

# ⌈ig-2 (PART 2)

```
               10         20         30         40         50         60
                !          !          !          !          !          !
    1 TCGACTTTCG TCAGGGGGGC TTTTATTTAT TCAATAATCC CTCCTCTCAA TAAATCTATT
      AGCTGAAAGC AGTCCCCCCG AAAATAAATA AGTTATTAGG GAGGAGAGTT ATTTAGATAA

   61 GTTGTACTTA ATTCAACTTC CATTTCTCTG TATCTTTCAA TACGCTCTTT TAAGTCCTTA
      CAACATGAAT TAAGTTGAAG GTAAAGAGAC ATAGAAAGTT ATGCGAGAAA ATTCAGGAAT

  121 ATTTCTTTTT TTAATTCCTC ATTTTCAGCA AATAACTCTT TTTCTTTGTT TGTCATTTTA
      TAAAGAAAAA AATTAAGGAG TAAAAGTCGT TTATTGAGAA AAAGAAACAA ACAGTAAAAT

  181 TTTCCCCCGT TTCAGCATCA AGAACCTTTG CATAACTTGC TCTATATCCA CACTGATAAT
      AAAGGGGGCA AAGTCGTAGT TCTTGGAAAC GTATTGAACG AGATATAGGT GTGACTATTA

  241 TGCCCTCAAA CCATAATCTA AAGGCGCTAG AGTTTGTTGA AACAATATCT TTTACATCAT
      ACGGGAGTTT GGTATTAGAT TTCCGCGATC TCAAACAACT TTGTTATAGA AAATGTAGTA

  301 TCGTATTTAA AATTCCAAAC TCCGCTCCCC TAAGGCGAAT AAAAGCCATT AAATCTTTTG
      AGCATAAATT TTAAGGTTTG AGGCGAGGGG ATTCCGCTTA TTTTCGGTAA TTTAGAAAAC

  361 TATTTACCAA ATTATAGTCA TCCACTATAT CTAAAAGTAA ATTCTTCAAT TCTCTTTTTT
      ATAAATGGTT TAATATCAGT AGGTGATATA GATTTTCATT TAAGAAGTTA AGAGAAAAAA

  421 GGCTTTCATC AAGTGTTATA TAGCGGTCAA TATCAAAATC ATTAATGTTC AAAATATCTT
      CCGAAAGTAG TTCACAATAT ATCGCCAGTT ATAGTTTTAG TAATTACAAG TTTTATAGAA

  481 TTTTGTCGTA TATATGTTTA TTCTTAGCAA TAGCGTCCTT TGATTCATGA GTCAAATATT
      AAAACAGCAT ATATACAAAT AAGAATCGTT ATCGCAGGAA ACTAAGTACT CAGTTTATAA

  541 CATATGAACC TTTGATATAA TCAAGTATCT CAACATGAGC AACTGAACTA TTCCCCAATT
      GTATACTTGG AAACTATATT AGTTCATAGA GTTGTACTCG TTGACTTGAT AAGGGGTTAA

  601 TTCGCTTAAT CTTGTTCCTA ACGCTTTCTA TTGTTACAGG ATTTCGTGCA ATATATATAA
      AAGCGAATTA GAACAAGGAT TGCGAAAGAT AACAATGTCC TAAAGCACGT TATATATATT

  661 CGTGATAGTG TGGTTTTTTA TAGTGCTTTC CATTTCGTAT AACATCACTA CTATTCCATG
      GCACTATCAC ACCAAAAAAT ATCACGAAAG GTAAAGCATA TTGTAGTGAT GATAAGGTAC

  721 TATCTTTATC TTTTTTTTCG TCCATATCGT GTAAAGGACT GACAGCCATA GATACGCCCA
      ATAGAAATAG AAAAAAAAGC AGGTATAGCA CATTTCCTGA CTGTCGGTAT CTATGCGGGT

  781 AACTCTCTAA TTTTTCTTTC CAATCATTAG GAATTGAGTC AGGATATAAT AAAAATCCAA
      TTGAGAGATT AAAAGAAAAG GTTAGTAATC CTTAACTCAG TCCTATATTA TTTTTAGGTT

  841 AGTTTCTAGC TTTAGTATTT AATAGCCATG ATATATTACC TTATCAAAAA CAAGTAGCGA
      TTAAAGATCG AAATCATAAA TTATCGGTAC TATATAATGG AATAGTTTTT GTTCATCGCT

  901 AAACTCGTAT CCTTCTAAAA ACGCGAGCTT TCGCTTATTT TTTTTATTCT GATTCCTTTC
      TTTGAGCATA GGAAGATTTT TGCGCTCGAA AGCGAATAAA AAAAATAAGA CTAAGGAAAG

  961 TTGCATATTC TTCTATAGCT AACGCCGCAA CCGCAGATTT TGAAAAACCT TTTTGTTTCG
      AACGTATAAG AAGATATCGA TTGCGGCGTT GGCGTCTAAA ACTTTTTGGA AAAACAAAGC

 1021 CCATATCTGT TAATTTTTTA TCTTGCTCTT TTGTCAGAGA AATCATAACT CTTTTTTTCG
      GGTATAGACA ATTAAAAAAT AGAACGAGAA AACAGTCTCT TTAGTATTGA GAAAAAAAGC

 1081 ATTCTGAAAT CACCATTTAA AAAACTCCAA TCAAATAATT TTATAAAATT AGTGTATCAC
      TAAGACTTTA GTGGTAAATT TTTTGAGGTT AGTTTATTAA AATATTTTAA TCACATAGTG

 1141 TTTGTAATCA TAAAAACAAC AATAAAGCTA CTTAAATATA GATTTATAAA AAACGTTGGC
      AAACATTAGT ATTTTTGTTG TTATTTCGAT GAATTTATAT CTAAATATTT TTTGCAACCG
```

19

# Fig-2 (PART 3)

```
1201 GAAAACGTTG GCGATTCGTT GGCGATTGAA AAACCCCTCA AACCCTTGAG CCAGTTGGGA
     CTTTTGCAAC CGCTAAGCAA CCGCTAACTT TTGGGGAGT TTGGGAACTC GGTCAACCCT

1261 TAGAGCGTTT TTGGCACAAA AATTGGCACT CGGCACTTAA TGGGGGGTCG TAGTACGGAA
     ATCTCGCAAA AACCGTGTTT TTAACCGTGA GCCGTGAATT ACCCCCCAGC ATCATGCCTT

1321 GCAAAATTCG CTTCCTTTCC CCCCATTTTT TTCCAAATTC CAAATTTTTT TCAAAAATTT
     CGTTTTAAGC GAAGGAAAGG GGGGTAAAAA AAGGTTTAAG GTTTAAAAAA AGTTTTTAAA

1381 TTCCAGCGCT ACCGCTCGGC AAAATTGCAA GCAATTTTTA AAATCAAACC CATGAGGGAA
     AAGGTCGCGA TGGCGAGCCG TTTTAACGTT CGTTAAAAAT TTTAGTTTGG GTACTCCCTT

1441 TTTCATTCCC TCAAACTCCC TTGAGCCTCC TCCAACCGAA ATAGAAGGAC GCTGCGCTTA
     AAAGTAAGGG AGTTTGAGGG AACTCGGAGG AGGTTGGCTT TATCTTCCTG CGACGCGAAT

1501 TTATTTCATT CAGTCATCGG CTTTCATAAT CTAACAGACA ACATCTTCGC TGCAAAGCAC
     AATAAAGTAA GTCAGTAGCC GAAAGTATTA GATTGTCTGT TGTAGAAGCG ACGTTTCGTG

1561 GCTACGCTCA AGGGCTTTTA CGCTACGATA ACGCCTGTTT TAACGATTAT GCCGATAACT
     CGATGCGAGT TCCCGAAAAT GCGATGCTAT TGCGGACAAA ATTGCTAATA CGGCTATTGA

1621 AAACGAAATA AACGCTAAAA CGTCTCAGAA ACGATTTTGA GACGTTTTAA TAAAAAATCG
     TTTGCTTTAT TTGCGATTTT GCAGAGTCTT TGCTAAAACT CTGCAAAATT ATTTTTTAGC

1681 ATAAATTATA TTGGCAAATT ATAAAAATGC CGTAGAACGA AAAATAGAGC CCTTAAAAAG
     TATTTAATAT AACCGTTTAA TATTTTTACG GCATCTTGCT TTTTATCTCG GGAATTTTTC

1741 CAAATAAACT ACGATAGCAA CAAAAAACAA AACCAAAACC CAAAAGATGA AAAATAAATA
     GTTTATTTGA TGCTATCGTT GTTTTTTGTT TTGGTTTTGG GTTTTCTACT TTTTATTTAT

1801 CAACAGCAAA AATAAATCAT AGTTCGATTT TTCGAACAAT GCGCACTTAC ACACCACTCC
     GTTGTCGTTT TTATTTAGTA TCAAGCTAAA AAGCTTGTTA CGCGTGAATG TGTGGTGAGG

1861 AAAATTGGGT GGTTTTTGTG CTTAAAAAAA TGTATCAGAA GTCGGCTAGC CGACAACAAA
     TTTTAACCCA CCAAAAACAC GAATTTTTTT ACATAGTCTT CAGCCGATCG GCTGTTGTTT

1921 AAAAGCGCTA ATAAATTAGC GCCATAAATA AAATATAAAT TAATCACTTT TTCTTTTTTT
     TTTTCGCGAT TATTTAATCG CGGTATTTAT TTTATATTTA ATTAGTGAAA AAGAAAAAAA

1981 GCCAAACAGC ATCGATCATT TTGTTTATTG CAATCGTATT GCTATTAATC GCAACATCAA
     CGGTTTGTCG TAGCTAGTAA AACAAATAAC GTTAGCATAA CGATAATTAG CGTTGTAGTT

2041 ACCAAAATAA AAGCCCCCT
     TGGTTTTATT TTCGGGGGA
```

Total number of bases is: 2059.
DNA sequence composition:    672 A;    424 C;    263 G;    700 T.

Sequence coded: NFSH71P.

# Fig-3

pSM76
6.6 kB

EcoRI
ClaI

Ap

EcoRI

P-b-GAL

HindIII

NaeI

NaeI

fSX

fSX = <u>Sal</u> I - <u>Xho</u> I FUSION

pNZ32
8.1 kB

SalI

SstI

EcoRI

P-b-GAL

HindIII

Km

Cm

BglII

fSN

fSN = <u>Sal</u> I - <u>Nae</u> I FUSION

# Fig-4

# fig-5

```
         10        20        30        40        50        60        70
         :         :         :         :         :         :         :
GGATCCAACTTGAAAAGGAACTGACTTATAAACTGATTTCAACAAACCGCAGACGAGTAGTTAAGGGTAATT

         80        90       100       110       120       130       140
         :         :         :         :         :         :         :
TATATTATGAACGTATAATCGTAGATAACCAGCTATTTAAGTTTGCAACTTTGAAAGATTTAATTGACTTGT

        150       160   HaeIII 170      180       190       200       210
         :         :         :         :         :         :         :
ATCACGAAAATATTAATGGGCCTATTTTTAGACAGAATCAGCTTCTTGTTAAAATGGGAGAGCAACCAATCG

        220       230       240       250       260       270       280
         :         :         :         :         :         :         :
AGGGTGGAGATATTTAACATAGCTAACCTTAACGCAGTTGCTGTTAAAAACCTAAGTGACCTACAAGGCAGT

   290       300       310       320       330       340       350       360
    :         :         :         :         :         :         :         :
AGAAAGGACGTAACAAGCACAGATGAAACTAATAACCAATAGTGCTGAAATTAAAGTAACTGAAAATGAGGA

                             METLysLeuIleThrAsnSerAlaGluIleLysValThrGluAsnGluAsp

        370       380       390       400       410       420       430
         :         :         :         :         :         :         :
CGGTTCTAAGTCGTTCCAAGGAATTGGTTCAGAAGTTGGTGTAGACAATCTTAACGGTATTGTCTTGACACC

         GlySerLysSerPheGlnGlyIleGlySerGluValGlyValAspAsnLeuAsnGlyIleValLeuThrPro

       440       450       460     .  470       480       490
        :         :         :         :         :         :
THACTGCATTGAGTTTGCTAGAGAACGATATCCATTGCTATATATGAACACGGAGCTGGATCC

        AsnCysIleGluPheAlaArgGluArgTyrProLeuLeuTyrMETAsnThrGluLeuAsp
```

# fig-7

pNZ36
7.5 kB

SalI
EcoRI
HindIII
BgIII
NsI